# EUROPEAN PATENT APPLICATION

(11) **EP 1 016 683 A1**
(43) Date of publication of application: **05.07.2000**
(21) Application number: 99926961.6
(22) Date of filing: 06.07.1999
(51) Int. Cl.: C08F 32/08, C08G 61/08, C07C 13/68, C07C 2/50

(54) **2-ETHYL-1,4,5,8-DIMETHANO-1,2,3,4,4a,5,8,8a-OCTAHYDRONAPHTHALENE COMPOSITION FOR POLYMERIZATION AND PROCESS FOR PRODUCING THE SAME**

(30) Priority: 06.07.1998 JP 20427098; 06.07.1998 JP 20427198
(71) Applicant: Nippon Petrochemicals Company, Limited, Tokyo 100-8530 (JP)
(72) Inventor: AIDA, Fuyuki, Yokohama-shi, Kanagawa 233-0007 (JP); SUZUKI, Takashi, Yokohama-shi, Kanagawa 235-0016 (JP); INOMATA, Yoshihisa, Yokohama-shi, Kanagawa 230-0021 (JP); MATSUMURA, Yasuo, Yokohama-shi, Kanagawa 245-0018 (JP)
(74) Representative: Strehl Schübel-Hopf & Partner
(86) International application number: JP9903652
(87) International publication number: WO0001743

(57) **Abstract**

A process for producing a 2-ethyl-1,4,5,8-dimethano-1,2,3,4,4a,5,8,8a-octahydronaphthalene composition in which the total content of two specific cyclopentadiene trimers is 100 to 5,000 ppm, which process comprises reacting cyclopentadiene and/or dicyclopentadiene with 5-ethyl-2-norbornene under specific conditions. By using the composition as a feed material for various kind of polymerization, homogeneous and transparent polymers containing no gel can be obtained.

## Description

### Technical Field

The present invention relates to a 2-ethyl-1,4,5,8-dimethano-1,2,3,4,4a,-5,8,8a-octahydronaphthalene composition for polymerization and process for producing the same, in which process cyclopentadiene and/or dicyclopentadiene and 5-ethyl-2-norbornene are caused to react.

### Background Art

Polymers and copolymers of cycloolefins have attracted considerable attention as polymeric materials that are excellent in an optical property, transparency; thermal resistance and oil-absorbing property. The cycloolefins, typically exemplified by tetracyclododecene, are useful as raw materials for producing such polymers.

These cycloolefins are generally prepared by polymerization using an organometallic complex catalyst. The mode of polymerization is roughly divided into two types. One process is homopolymerization at the olefin-site of cycloolefin or copolymerization with lower α-olefins using Ziegler catalyst or metallocene catalyst. The other process is metathesis polymerization using a carbene-type complex.

As a process for producing tetracyclododecenes, for example, the process as disclosed in U. S. Patent No. 4,320,239 is known. It is disclosed that in a process to prepare a mixture of norbornene and tetracyclododecene by heating α-olefin, cyclopentadiene or dicyclopentadiene and norbornene, the norbornene is recycled for reuse.

However, due to the nature of preparation method itself or other reasons, the obtained tetracyclododecene sometimes contains small amount of isomers and other compounds. There is an apprehension that some of these compounds may inhibit specific polymerization of tetracyclododecenes.

For instance, Japanese Laid-Open Patent Publication No. 7-173085 discloses a composition of tetracyclododecenes containing specific diolefin compounds, in which two norbornene rings have unsaturated double bonds, respectively. Because specific diolefin compound causes gelation in the metathesis polymerization of tetracyclododecene, it is proposed that the content of the above specific diolefin compound must be limited to the range of 50 to 5,000 ppm. The foregoing composition as disclosed in the above patent gazette particularly relates to the monomer for use in the ring opening metathesis polymerization. Meanwhile, homopolymerization at olefinic sites of cycloolefin and copolymerization of lower α-olefins using metallocene catalyst or Ziegler catalyst are not referred to. Furthermore, only the foregoing specific diolefin compounds are pointed out.

The object of the present invention is to carry out investigation on various polymerization processes of specific tetracyclododecenes and to find out a composition that is suitable for use in various kinds of polymerization and a method for producing the same.

### Disclosure of Invention

A first aspect of the present invention relates to 2-ethyl-1,4,5,8-dimethano-1,2,3,4,4a,5,8,8a-octahydronaphthalene composition for polymerization that is represented by the following formula [III], wherein the total content of cyclopentadiene trimers represented by the following formula [I] or [II] is in the range of 100 to 5,000 ppm.

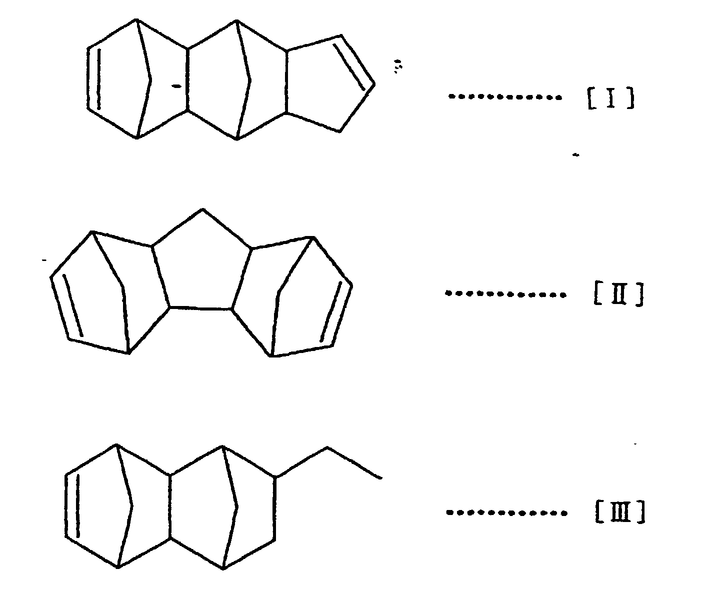

A second aspect of the present invention relates to a composition of 2-ethyl-1,4,5,8-dimethano-1,2,34,4a,5,8,8a-octahydronaphthalene for use in polymerization represented by the formula [III] as referred to in the above first aspect of the invention, in which the content of cyclopentadiene trimer as represented by the formula [II] is in the range of 100 to 3,000 ppm.

A third aspect of the present invention relates to a process for producing a composition of 2-ethyl-1,4,5,8-dimethano-1,2,3,4,4a,5,8,8a-octahydronaphthalene for polymerization represented by the above formula [III], in which composition the total content of cyclopentadiene trimers represented by the above formulae [I] and [II] are in the range of 100 to 5,000 ppm, and the process comprises the step of reacting cyclopentadiene and/or dicyclopentadiene and 5-ethyl-2-norbornene as represented by the following formula [IV] at temperature of 100 to 350°C, pressure of 0.5 to 20 MPa and residence time of 0.1 to 360 min., in which quantity of 5-ethyl-2-norbornene is 1 to 20 times by mole as much as the sum of moles of dicyclopentadiene plus one half of moles of cyclopentadiene.

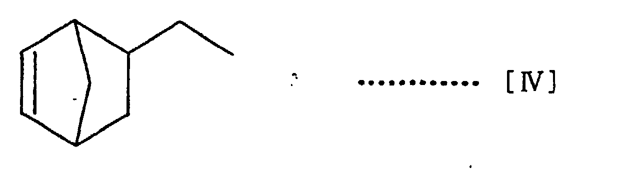

A fourth aspect of the present invention relates to a process for producing a composition of 2-ethyl-1,4,5,8-dimethano-1,2,3,4,4a,5,8,8a-octahydronaphthalene for polymerization represented by the above formula [III], in which composition the total content of cyclopentadiene trimers represented by the above formula [I] or [II] is in the range of 100 to 5,000 ppm, and the process comprises the step of reacting cyclopentadiene and/or dicyclopentadiene, 5-ethyl-2-norbornene as represented by the above formula [IV] and 1-butene at temperature of 100 to 350°C, pressure of 0.5 to 20 MPa and residential time of 0.1 to 360 min., in which the quantity of 1-butene is 0.1 to 10 times by mole and quantity of 5-ethyl-2-norbornene is 1 to 20 times by mole as much as the sum of moles of dicyclopentadiene plus one half of moles of cyclopentadiene.

A fifth aspect of the present invention relates to a process for producing a composition of 2-ethyl-1,4,5,8-dimethano-1,2,3,4,4a,5,8,8a-octahydronaphthalene for polymerization represented by the above formula [III], in which composition the total content of cyclopentadiene trimers represented by the above formula [I] or [II] is in the range of 100 to 5,000 ppm, and the process comprises the steps of: reacting cyclopentadiene and/or dicyclopentadiene, 5-ethyl-2-norbornene as represented by the above formula [IV] and 1-butene at temperature of 100 to 350°C, pressure of 0.5 to 20 MPa and residential time of 0.1 to 360 min. and reusing the 5-ethyl-2-norbornene by recycling, in which quantity of 1-butene is 0.1 to 10 times by mole and quantity of 5-ethyl-2-norbornene is 1 to 20 times by mole as much as the sum of moles of dicyclopentadiene plus one half of moles of cyclopentadiene.

A sixth aspect of the present invention relates to a process for producing a composition of 2-ethyl-1,4,5,8-dimethano-1,2,3,4,4a,5,8,8a-octahydronaphthalene for polymerization represented by the above formula [III] as described in the foregoing third to fifth aspects of the invention, in which content of cydopentadiene trimer represented by the above formula [II] is in the range of 100 to 3,000 ppm.

The present invention will be described in more detail.

The above cyclopentadiene trimers comprise diolefin compounds as represented by the formula [I], which contains a norbornene-type olefinic structure and a cyclopentene-type olefinic structure, and diolefin compounds as represented by the formula [II], which contains a pair of norbornene-type olefinic structure. Total content of these compounds is in the range of 100 to 5,000 ppm, preferably 100 to 3,000 ppm. When content of cyclopentadiene trimers exceeds the upper limit of these range, cross-linking reaction is liable to occur not only in ring opening metathesis polymerization but also in homopolymerization of cycloolefins at olefinic sites or cop olymerization with lower α-olefins. Thus, in the obtained polymers, polydispersed structure is notable and gelation is liable to occur. When content of cyclopentadiene trimers is less than the lower limit of the above-mentioned range, it is not economically preferable because costs for isolation and refining increase.

The preferable cyclopentadiene trimer is the one as represented by the formula [II], which contains a pair of norbornene-type olefinic structure. Content of this compound represented by the formula [II] is in the range of 100 to 3,000 ppm, preferably 100 to 2,000 ppm.

The first method for producing a composition of 2-ethyl-1,4,5,8-dimethano-1,2,3,4,4a,5,8,8a-octahydronaphthalene for use in polymerization as represented by the formula [III] in which total content of cydopentadiene trimers represented by the formulae [I] and [II] is in the range of 100 to 5,000 ppm, comprises the step of: reacting cyclopentadiene and/or dicyclopentadiene and 5-ethyl-2-norbornene as represented by the foregoing formula [IV] at temperature of 100 to 350°C, pressure of 0.5 to 20 MPa and residential time of 0.1 to 360 min., in which quantity of 5-ethyl-2-norbornene is 1 to 20 times by mole as much as sum of moles of dicyclopentadiene plus one half of moles of cyclopentadiene.

As the feed material of dicyclopentadiene, commercially available ones having a purity of desirably not less than 90% can be used. The impurities contained in dicyclopentadiene are mainly tetrahydromethylindene as well as the addition products of C₅ to C₉ chain or cyclic diolefins with cyclopentadiene. When content of these impurities is large, formation of high-boiling by-products increases. When cyclopentadiene is used, it is previously subjected to thermal-cracking distillation. Commercially available cyclopentadiene is in the form of dimer, so that cyclopentadiene is easily obtained by thermally cracking the obtained one. If reaction is carried out at a temperature above the thermal-cracking temperature, dicyclopentadiene can also be used as a feed material.

Concerning ratios of feed materials in the above method, moles of cyclopentadiene and/or dicyclopentadiene are represented on the basis of moles of dicyclopentadiene. For example, when a mixture of 2 moles of cyclopentadiene and 1 mole of dicyclopentadiene is used, it is regarded as 2 moles of dicyclopentadiene. Feed mole of 5-ethyl-2-norbornene is 1 to 20 times, preferably 2 to 10 times, and more preferably 3 to 8 times moles of thus obtained moles of dicyclopentadiene. If mole of 5-ethyl-2-norbornene is larger than the above range, formation of high-boiling compounds as typically represented by the formulae [I] and [II] is relatively low, while reducing the effective yield of the intended product. On the other hand, if it is less than this range, formation of high-boiling compounds of the formulae [I] and [II] increases.

5-Ethyl-2-norbornene as represented by the foregoing formula [IV] is not especially limited and those prepared in any method can be used. For example, it is synthesized from 1-butene and cyclopentadiene and/or dicyclopentadiene under the conditions of reaction temperature of 100 to 350°C and pressure of 0.1 to 20 MPa.

In other words, conditions of reaction temperature of 100 to 350°C and pressure of 0.1 to 20 MPa for producing 5-ethyl-2-norbornene from 1-butene and cyclopentadiene and/or dicyclopentadiene are coincident with those for preparation of 2-ethyl-1,4,5,8-dimethano-1,2,3,4,4a,5,8,8a-octahydronaphthalene represented by the formula [III].

Accordingly, when cyclopentadiene and/or dicyclopentadiene and 5-ethyl-2-norbornene are mixed and heated, if 1-butene is also added, the intended product of the present invention is also produced together with 5-ethyl-2-norbornene. Therefore, it is quite convenient with coexistence of 1-butnene because 2-ethyl-1,4,5,8-dimethano-1,2,3,4,4a,5,8,8a-octahydronaphthalene composition for polymerization is produced simultaneously with the preparation of 5-ethyl-2-norbornene. In other words, by employing this method, it is not necessary to synthesize separately or purchase 5-ethyl-2-norbornene.

The second method for producing a composition of 2-ethyl-1,4,5,8-dimethano-1,2,3,4,4a,5,8,8a-octahydronaphthalene comprises the step of reacting cyclopentadiene and/or dicyclopentadiene, 5-ethyl-2-norbornene as represented by the foregoing formula [IV] and 1-butene at temperature of 100 to 350°C, pressure of 0.5 to 20 MPa and residential time of 0.1 to 360 min., in which quantity of 1-butenen is 0.1 to 10 times by mole and quantity of 5-ethyl-2-norbornene is 1 to 20 times by mole as much as sum of moles of dicyclopentadiene plus one half of moles of cyclopentadiene.

Concerning ratios of feed materials in the above second method, moles of cyclopentadiene and/or dicyclopentadiene are represented on basis of moles of dicyclopentadiene. The feed mole of 1-butene is 0.1 to 10 times, preferably 0.2 to 8 times and more preferably 0.5 to 5 times as much as the above defined mole value. 5-Ethyl-2-norbornene is 1 to 20 times, preferably 2 to 10 times, and more preferably 3 to 8 times moles of the thus obtained moles of dicyclopentadiene. If quantity of 1-butene is smaller than the above range, formation of high-boiling compounds increases. On the other hand, if it is larger than this range, formation of high-boiling compounds decreases, however, effective yield of intended product decreases.

The feed mole of 5-ethyl-2-norbornene is 1 to 20 times, preferably 2 to 10 times, and more preferably 3 to 8 times moles of dicyclopentadiene. If quantity of 5-ethyl-2-norbornene is larger than the above range, formation of high-boiling compounds as represented by the formulae [I] and [II] is relatively low, however, effective yield of the intended product decreases. On the other hand, if it is less than the above range, formation of high-boiling compounds of the formulae [I] and [II] increases.

1-Butene that can be used in the above process is exemplified by various products obtained from petroleum refinery plants, several cracking processes for butane, naphtha or light oil, reaction product in disproportionation of propylene, and reaction product of dimerization of ethylene.

Furthermore, 5-ethyl-2-norbornene is produced in the processes done under the coexistence of 1-butene, so that at least a part of the 5-ethyl-2-norbornene is recovered by separation and it can be recycled for reuse. Especially, if the reaction is carried out at least under the condition that the quantity of 5-ethyl-2-norbornene is not reduced, it is desirable to produce 2-ethyl-1,4,5,8-dimethano-1,2,3,4,4a,5,8,8a-octahydronaphthalene from 1-butene and cydopentadiene and/or dicyclo-pentadiene without replenishing the reaction system with 5-ethyl-2-norbornene by recycling it.

Each component of the present invention such as cyclopentadiene and/or dicyclopentadiene can be supplied to a reaction system by any method. In continuous production of 2-ethyl-1,4,5,8-dimethano-1,2,3,4,4a,5,8,8a-octahydronaphthalene, it is desirable to use a booster and/or pump. It may be previously mixed with another material of 5-ethyl-2-norbornene or they can be fed separately. When they are fed separately, two storage tanks and two pumps are necessary; so that it is preferable to mix them beforehand.

In the present invention, several solvents can be used. As the solvents, any of paraffinic, naphthenic and aromatic hydrocarbons can be used. It is convenient to use a solvent, which can be recovered simultaneously with 5-ethyl-2-norbornene. In other words, a solvent having a boiling point dose to that of 5-ethyl-2-norbornene, is suitable. The number of carbon atoms of the solvent is preferably from 6 to 13. Among them, there are exemplified by commonly used solvents such as normal hexane, isohexane, benzene, heptane, isoheptane, toluene, normal octane, isooctane, dimethylcyclohexane, ethylcyclohexane, xylene, ethylbenzene, trimethylbenzene, methylcyclohexane, tetrahydronaphthalene, and decahydronaphthalene. The ratio of addition may preferably be 300% by weight or less relative to 5-ethyl-2-norbornene.

Incidentally, yield of 2-ethyl-1,4,5,8-dimethano-1,2,3,4,4a,5,8,8a-octahydronaphthalene can also be improved with the use of a solvent.

Synthesis of 2-ethyl-1,4,5,8-dimethano-1,2,3,4,4a,5,8,8a-octahydronaphthalene can be carried out using these compounds as raw materials, and if necessary, with a solvent. The type of reaction may be batchwise or continuous. When batchwise reaction is selected, a perfectly mixing type reactor is employed. In the case of continuous reaction, any of continuous stirred isothermal reactor and plug flow tubular reactor can be used. Examples of commercially available plug flow reactors include "Static Mixer" made by Noritake Co., Ltd.; "Sulzer Mixer" made by Sumitomo Heavy Industry Ltd.; and "Square Mixer" made by Sakura Seisakusho, Ltd. Reaction can be carried out either in a single stage system or a multi-stage system having two or more stages. In the case of multistage system, it is also possible to use continuous stirred isothermal reactors or plug flow tubular reactors by connecting them in parallel or in series.

Concerning reaction conditions in continuous type reaction and batchwise reaction, the reaction pressure is in the range of 0.1 to 50 MPa, preferably 0.5 to 40 MPa and more preferably 1 to 10 MPa. The reaction temperature is in the range of 100 to 350°C, preferably 100 to 330°C and more preferably 200 to 300°C. Especially, when dicyclopentadiene is used as a feed material, the reaction temperature must be 100°C or above, otherwise decomposition into cyclopentadiene is not caused. When the reaction temperature is higher, the formation of high-boiling compounds represented by the formulae [I] and [II] is liable to increase.

The space velocity in continuous system is in the range of 0.001 to 100 h⁻¹, preferably 0.1 to 50 h⁻¹, and more preferably 1 to 10 h⁻¹. When space velocity exceeds 100 h⁻¹, it is not suitable because of increasing unreacted materials.

The suitable residential time in a batchwise system is in the range of 0.1 to 360 min. When the residential time is longer than this range, much high-boiling by-products are formed.

The reaction mixture taken out from the reactor is introduced into a distillation process. The components of recycling 5-ethyl-2-norbornene, solvent (when used) and 2-ethyl-1,4,5,8-dimethano-1,2,3,4,4a,5,8,8a-octahydronaphthalene are separated and refined. Distillation is preferably employed for the refining and distillation may be any of batchwise and continuous.

In the case of batchwise distillation process, the reaction mixture sometimes contains unreacted 1-butene and cyclopentadiene, so that they are separated at first. Concerning the distillation conditions, pressure in the range of 0.1 to 1 MPa and temperature in the range of 0 to 100°C are optionally selected. In the next step, 5-ethyl-2-norbornene is recovered. Especially, when a solvent is used, they are simultaneously recovered. As the distillation conditions, pressure in the range of 1 to 100 kPa and temperature in the range of 20 to 140°C are suitable. If necessary the concentration of the mixture of 5-ethyl-2-norbornene and solvent is adjusted and it is reused as a feed material. In the subsequent step, the remained dicyclopentadiene is then recovered at temperature in the range of 40 to 170°C and pressure in the range of 1 to 100 kPa. When dicyclopentadiene does not remain, this distillation step is not necessary However, if impurities of dicyclopentadiene-origin exist, it is desirable to carry out the elimination of them under the above conditions. Through this operation, purity of 2-ethyl-1,4,5,8-dimethano-1,2,3,4,4a,5,8,8a-octahydronaphthalene can be maintained high. The subsequent distillation of 2-ethyl-1,4,5,8-dimethano-1,2,3,4,4a,5,8,8a-octahydronaphthalene is carried out under the conditions of temperature in the range of 40 to 160°C and pressure in the range of 0.01 to 30 kPa.

In the case of continuous distillation process, it is preferable to employ at least two distillation columns. When two distillation columns are used, 1-butene, cyclopentadiene, 5-ethyl-2-norbornene, solvent (when used), and residual dicyclopentadiene are recovered from the top of the first column and the mixture at the bottom of the column is introduced into the second column. The intended product of 2-ethyl-1,4,5,8-dimethano-1,2,3,4,4a,5,8,8a-octahydronaphthalene is then taken out from the top of the second column, while the high-boiling substances are obtained from the bottom of the second column.

In this case, as for the distillation conditions in the first column, temperature in the range of 20 to 173°C and pressure in the range of 1 to 100 kPa are suitable when dicyclopentadiene still remains. When it does not remain, pressure in the range of 1 to 100 kPa and temperature in the range of 35 to 140°C are suitable.

As the distillation conditions in the second column, temperature of 40 to 160°C and pressure of 0.01 to 30 kPa are employed. When dicyclopentadiene is absent, 1-butene, cyclopentadiene, 5-ethyl-2-norbornene and solvent (when used) are recovered under the conditions of pressure of 1 to 100 kPa and temperature of 30 to 140°C. When the dicyclopentadiene contains impurities, it is preferable to carry out an additional operation to remove them under the conditions of temperature of 20 to 173°C and pressure of 1 to 100 kPa. Through this process, it is possible to maintain the high purity of 2-ethyl-1,4,5,8-dimethano-1,2,3,-4,4a,5,8,8a-octahydronaphthalene.

Meanwhile, the content of impurities such as tetrahydromethylindene having a very high decomposition point is large in the recovered dicyclopentadiene. If these impurities are mixed in 5-ethyl-2-norbornene, they are accumulated during the recycling use of the norbornene and the formation of by-product increases. When the reaction is done at higher temperature in order to accelerate the decomposition, much by-product is formed. Therefore, the process using two distillation column is employed only when the dicyclopentadiene does not exist or hardly exists in the reaction product.

When three distillation columns are used, 1-butene and cyclopentadiene are mainly separated from the top of first column. The distillation conditions are arbitrarily selected from the ranges of pressure of 0.1 to 1 MPa and temperature of 0 to 100°C.

The mixture obtained from the bottom of the first column is introduced into the second column. 5-Ethyl-2-norbornene, solvent (when used) and, as occasion demands, residual dicyclopentadiene are simultaneously recovered from the top of the second column. When dicyclopentadiene remains, temperature of 20 to 173°C and pressure of 1 to 100 kPa are suitable as the distillation conditions. Meanwhile, if it does not remain, pressure of 1 to 100 kPa and temperature of 35 to 140°C are suitable.

The mixture obtained from the bottom of the second column is introduced into the third column. 2-Ethyl-1,4,5,8-dimethano-1,2,3,4,4a,5,8,8a-octahydronaphthalene is separated from the top of the third column under the conditions of temperature of 40 to 160°C and pressure of 0.01 to 30 kPa. When no dicyclopentadiene remains, 5-ethyl-2-norbornene and solvent (when used) are recovered under the distillation conditions of pressure of 1 to 100 kPa and temperature of 20 to 140°C. When dicydlopentadiene contains impurities, however, it is preferable to perform an additional removal operation under the conditions of temperature of 50 to 100°C and pressure of 20 kPa or lower. Through these operations, it is possible to maintain purity of 2-ethyl-1,4,5,8-dimethano-1,2,3,4,4a,5,8,8a-octahydronaphthalene on a high level.

When four distillation columns are used, 1-butene and cyclopentadiene are separated from the top of the first column. The distillation conditions can be arbitrarily selected from the range of pressure of 0.1 to 1 MPa and temperature of 0 to 100°C.

The mixture obtained from the bottom of column is introduced into the second column, and 5-ethyl-2-norbornene and solvent (when used) are recovered simultaneously from the top of the column. The distillation conditions employed are pressure of 1 to 100 kPa and temperature of 35 to 140°C.

The mixture obtained from the bottom of second column is introduced into the third column, and the mixture mainly composed of dicyclopentadiene is separated. As the distillation conditions, temperature of 40 to 173°C and pressure of 1 to 100 kPa are suitable.

Meanwhile, the content of impurities such as tetrahydromethylindene having a very high decomposition point is increased in the recovered dicyclopentadiene. If these impurities are mixed into 5-ethyl-2-norbornene, they are accumulated during the recycling use of the norbornene and the formation of by-product increases. Furthermore, when the reaction is done at higher temperature in order to accelerate the decomposition, the formation of by-products may increase. Therefore, mixing of impurities into the 5-ethyl-2-norbornene is preferably avoided as small as possible because accumulation of dicyclopentadiene occurs.

The mixture obtained from the bottom of the third column is introduced into the fourth column so as to separate 2-ethyl-1,4,5,8-dimethano-1,2,3,4,4a,-5,8,8a-octahydronaphthalene under the conditions of temperature of 40 to 160°C and pressure of 0.01 to 30 kPa, while high-boiling compounds as by-products are obtained from the bottom of the column.

In another method employing four distillation columns, 1-butene and cydopentadiene are separated from the top of first column. As the distillation conditions in this case, pressure in the range of 0.1 to 1 MPa and temperature in the range of 0 to 100°C are optionally selected.

The mixture obtained from the bottom of this column is introduced into the second column, and 5-ethyl-2-norbornene and dicydopentadiene are recovered from the top of the column. The distillation conditions of temperature of 20 to 173°C and pressure of 1 to 100 kPa are employed.

The mixture obtained from the top of the second column is introduced into the third column, and 5-ethyl-2-norbornene is separated from the top of the column and dicyclopentadiene is separated from the bottom of the column. As distillation conditions for the third column, pressure of 1 to 100 kPa and temperature of 35 to 140°C are suitable.

Meanwhile, the content of impurities such as tetrahydromethylindene having a very high decomposition point is large in the recovered dicyclopentadiene. If these impurities are mixed into 5-ethyl-2-norbornene, they are accumulated in norbornene during the recycling use of the norbornene and the formation of by-product increases. Furthermore, when the reaction is done at higher temperature in order to accelerate the decomposition, much by-product is formed. Therefore, the content of the by-product in 5-ethyl-2-norbornene must be avoided as small as possible.

The mixture obtained from the bottom of the second column is introduced into the fourth column. 2-Ethyl-1,4,5,8-dimethano-1,2,3,4,4a,5,8,8a-octahydronaphthalene is separated from the top of the column under the conditions of temperature of 40 to 160°C and pressure of 0.01 to 30 kPa. The by-products of high boiling substances are obtained from the bottom of the column.

In either batch operation or continuous operation, it is important to conduct the distillation while maintaining the maximum temperature of 200°C or below because the 2-ethyl-1,4,5,8-dimethano-1,2,3,4,4a,5,8,8a-octahydronaphthalene is liable to be decomposed at higher temperatures.

In order to enhance the separation efficiency, it is possible to fill each distillation column with various kinds of packing materials or to carry out refluxing. The theoretical number of plates of each distillation column is from 1 to 100, preferably from 2 to 50, and more preferably from 3 to 30. The suitable reflux ratio is determined according to the condition of separation in each distillation column and ratio in the range of 1 to 50 is suitable.

The thus recovered 5-ethyl-2-norbornene or the mixture of 5-ethyl-2-norbornene and solvent is returned to a feedstock tank for reuse by recycling. The 5-ethyl-2-norbornene and/or solvent that are lost as exhaust gases during the distillation, can be optionally supplemented from the feedstock tank. The norbornene, which has increased during the reaction, may be discharged in order to maintain the concentration at a certain level.

Furthermore, in the reaction of the present invention, it is possible to add optionally antioxidants, polymerization inhibitors or the like.

The composition of 2-ethyl-1,4,5,8-dimethano-1,2,3,4,4a,5,8,8a-octahydronaphthalene that is produced through the foregoing method, can be used for the foregoing known processes such as metathesis polymerization or homopolymerization at olefinic sites or copolymerization with α-olefin.

Metathesis polymerization can be carried out according to the methods as disclosed, for example, in Japanese Laid-Open Patent Publication Nos. 9-183832 and 8-151435, U. S. Patent Nos. 5,462,995 and 5,580,934, Japanese Laid-Open Patent Publication Nos. 1-168725, 1-168724 and 60-026024. More particularly, these methods are done using catalysts comprising (a) catalyst components of transition metal compounds and (b) catalyst promoters of metal compounds.

The catalyst components of transition-metal compounds (a) are the those of transition metals belonging to Groups IVB, VB, VIB, VIIB or VIII of the periodic table, and more particularly, there are exemplified by halides, oxyhalides, alkoxyhalides, alkoxides, carboxylates, (oxy)acetylacetonates, carbonyl complexes, acetonitrile complexes, hydride complexes, derivatives of them, and complexes of phosphine compounds of them.

The catalyst promoters of metallic compounds (b) are compounds of metals belonging to Groups IA, IIA, IIB IIIA, and IVA of the periodic table, each having at least one metal-carbon bond or metal-hydrogen bond such as organic compounds of Al, Sn, Li, Na, Mg, Zn, Cd and B.

Furthermore, in addition to both the above components (a) and (b), in order to enhance activity in metathesis polymerization, it is possible to add aliphatic tertiary amine, aromatic tertiary amine, molecular oxygen, alcohol, ethers, peroxide, carboxylic add, acid anhydride, add chloride, ester, ketone, nitrogen-containing compounds, sulfur-containing compounds, halogen-containing compounds, molecular iodine, or Lewis acid.

Meanwhile, homopolymerization at olefinic sites of tetracyclodecene or copolymerization with lower α-olefin can be carried out using metallocene catalyst or Ziegler catalyst. When metallocene catalyst is used, the methods as disclosed, for example, in U.S. Patent No. 5,087,677, Japanese Laid-Open Patent Publication No. 2-173112, U.S. Patent No. 5,344,900, European Patent No. 0283164 A, and Japanese Laid-Open Patent Publication No. 61-221206, can be employed. More particularly, there are methods using a catalyst consisting of a component (c) of a compound of transition metal belonging to Group IVB, VB or VIB of the periodic table and a component (b) of organoaluminum oxy compound.

The component (c) of transition metal compound is the one of a transition metal belonging to Groups IVB, VB or VIB of the periodic table. This type of compounds have at least two cycloalkadienyl groups or their substituted compounds, these cycloalkadienyl groups or their substituted compounds are bonded through hydrocarbon groups or silylene groups or substituted silylene groups, so called multidentate ligands.

The component (d) of organoaluminum oxy compound is a compound represented by the following formulae [V] and [VI]. In these formulae, R denotes a hydrocarbon group, more particularly, methyl, ethyl, propyl, and butyl, and m is an integer of 2 or more.

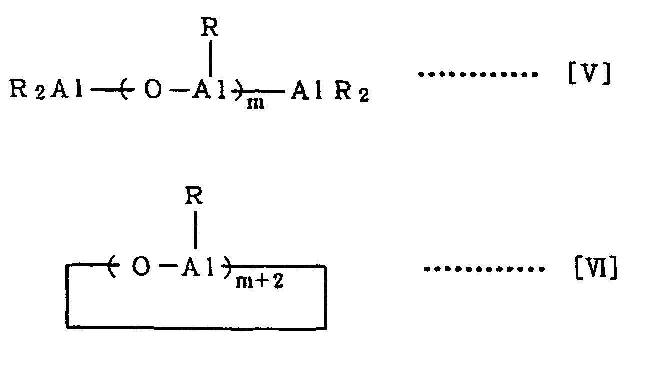

Titanium compounds or vanadium compounds are used for Ziegler catalyst. For example, as disclosed in Japanese Laid-Open Patent Publication No. 9-176396 and European Patent No. 0203799 A, a copolymer of cycloolefin with α-olefin such as ethylene can be synthesized using a catalyst formed of organoaluminum compound and vanadium compound that is soluble in a hydrocarbon solvent.

The 2-ethyl-1,4,5,8-dimethano-1,2,3,4,4a,5,8,8a-octahydronaphthalene according to the present invention can be copolymerized with other olefinic compounds such as ethylene, norbornene and dicyclopentadiene. Furthermore, as disclosed in U. S. Patent No. 5,191,026, the balances among various physical properties can be adjusted by changing the proportion of copolymerization materials.

The polymer obtained by the above ring opening polymerization sometimes contains olefinic double bonds. If heat resistance and light resistance of the resultant polymer are poor, a part of or all of the double bonds are hydrogenated in order to improve these properties. Hydrogenation is carried out using a catalyst of nickel or a precious metal of palladium or platinum that is dispersed and supported on an inorganic carrier. Furthermore, homogeneous hydrogenation catalyst can also be used. As the hydrogenation conditions, pressure in the range of 0.1 to 20 MPa and temperature in the range of 0 to 200°C are employed.

### Best Method to Carry Out the Embodiment of the Invention

The present invention will be described in more detail with reference to the following examples.

### 〈Example 1〉

2,000 g of dicyclopentadiene (94.9% purity) and 2,240 g of 1-butene were introduced into 10 lit. autoclave and they were allowed to react at 230°C for 3 hours. The reaction mixture was subjected to fractional distillation to obtain 3,400 g of 5-ethyl-2-norbornene.

Using a tubular reactor of 5 mm in inner diameter, a portion of 127 cm having inner volume of 25 ml was immersed into an oil bath, which was regulated to 250°C. 3,400 g of the 5-ethyl-2-norbornene synthesized in the above step and 460 g of dicyclopentadiene were mixed together and the mixture was fed into the tubular reactor using a pump at space velocity of 4 h⁻¹. At a point before the heating zone, 1-butene was continuously fed, in which the molar ratio of 1-butene to dicyclopentadiene was adjusted to 1:1. The pressure in the reaction system was maintained to 5 MPa using a pressure-regulating valve. The reaction was continued for 10 hours to obtain a reaction mixture and it was subjected to fractional distillation under pressure of 0.1 kPa to obtain 108 g of a fraction mainly containing 2-ethyl-1,4,5,8-dimethano-1,2,3,4,4a,5,8,8a-octahydronaphthalene. As a result, 3,650 ppm of the compound represented by the formula [I] and 1,250 ppm of the compound represented by the formula [II] were contained in this fraction.

### 〈Polymerization Example 1〉

Into a 2 lit. round-bottle flask equipped with stirring blades was introduced 1,000 ml of toluene that was dried under nitrogen atmosphere. To this flask were then added 1 mmol of dichloroethoxyoxo vanadium and 10g of the 2-ethyl-1,4,5,8-dimethano-1,2,3,4,4a,5,8,8a-octahydronaphthalene that was synthesized in Example 1, containing 3,650 ppm of the compound represented by the formula [I] and 1,250 ppm of the compound represented by the formula [II]. This solution was subjected to bubbling with stirring and with supplying a gas mixture of ethylene and nitrogen in the ethylene/nitrogen molar ratio of 1:5 at a flow rate of 200 lit./hr. Polymerization was started with further adding dropwise 10 mmol of ethylaluminum sesquichloride and was continued for 20 min at 5°C.

Polymerization was terminated by adding 30 ml of methanol and the copolymer was precipitated by pouring the reaction mixture into 2 lit. of methanol. The obtained copolymer was dried under reduced pressure and molded into a pressed sheet of 1 mm thickness at 240°C. The thus obtained sheet was transparent and no gelation was observed.

### 〈Comparative Example 1〉

A similar test as in Example 1 was carried out except that the synthesis of 2-ethyl-1,4,5,8-dimethano-1,2,3,4,4a,5,8,8a-octahydronaphthalene was carried out at 380°C. 92 g of a fraction mainly containing 2-ethyl-1,4,5,8-dimethano-1,2,3,4,4a,5,8,8a-octahydronaphthalene was obtained. This fraction contained 5,300 ppm of the compound represented by the formula [I] and 3,500 ppm of the compound represented by the formula [II].

### 〈Polymerization Example 2〉

Copolymerization with ethylene was carried out in the like manner as m Polymerization Example 1 except that the 2-ethyl-1,4,5,8-dimethano-1,2,3,4,-4a,5,8,8a-octahydronaphthalene containing 5,300 ppm of the compound represented by the formula [I] and 3,500 ppm of the compound represented by the formula [II] as prepared in Comparative Example 1 was used. The obtained copolymer was molded into a pressed sheet.

Gel was observed in the sheet. This gel was cut off and heated up to 300°C on a hot plate, however, it did not melt.

### 〈Polymerization Example 3〉

To a thoroughly dried 500 ml autoclave were added 20g of the 2-ethyl-1,4,5,8-dimethano-1,2,3,4,4a,5,8,8a-octahydronaphthalene containing 3,650 ppm of the compound represented by the formula [I] and 1,250 ppm of the compound represented by the formula [II] as synthesized in Example 1; 2.5 g of norbornene, 2.5 g of dicyclopentadiene and 200 ml of toluene. Then, 3 mmol of triethyl aluminum was added to the mixture, and furthermore, 0.7 mmol of titanium tetrachloride and 7 mmol of triethylamine were added and ring opening polymerization was carried out at 30°C for 6 hours. The polymer was precipitated by pouring the reaction mixture into a mixture of acetone and isopropyl alcohol and the obtained polymer was dissolved again in toluene. This operation of precipitation was repeated twice and the precipitate was dried under reduced pressure to obtain 21g of a polymer.

Into a 200ml autoclave were added 0.4g of 0.5% palladium-carbon and 100 ml of toluene and 4g of the polymer obtained by the ring opening polymerization was further added. Hydrogenation was carried out at 20°C for 1 hour under hydrogen pressure of 5 MPa. After the reaction, the catalyst was removed by filtration and re-precipitation was done using methanol.

The obtained copolymer was dried under reduced pressure and molded into a pressed sheet of 1 mm thickness at 350°C. The sheet was homogeneous and transparent and any gelation was not observed.

### 〈Polymerization Example 4〉

Polymerization and hydrogenation were carried out in the like manner as in Polymerization Example 3 except that the used feed material was the 2-ethyl-1,4,5,8-dimethano-1,2,3,4,4a,5,8,8a-octahydronaphthalene containing 5,300 ppm of the compound represented by the formula [I] and 3,500 ppm of the compound represented by the formula [II] that was synthesized in Comparative Example 1 to obtain a polymer. The obtained polymer was molded into a pressed sheet, gel was observed.

### 〈Example 2〉

Using a tubular reactor of 5 mm in inner diameter, a portion of 127 cm having inner volume of 25 ml was immersed into an oil bath, which was regulated to 250°C. 3,400 g of 5-ethyl-2-norbornene, 460 g of dicyclopentadiene and 500 g of methylcyclohexane were mixed together and the mixture was fed into the tubular reactor using a pump at space velocity of 4 h⁻¹. At a point before the heating zone, 1-butene was continuously fed, in which the molar ratio of 1-butene to dicyclopentadiene was adjusted to 1:1. The pressure in the reaction system was maintained to 5 MPa using a pressure-regulating valve. The reaction was continued for 10 hours to obtain a reaction mixture and it was subjected to fractional distillation at 0.1 kPa. 600 g of 5-ethyl-2-norbornene was recovered and 106 g of a fraction of intended product mainly containing 2-ethyl-1,4,5,8-dimethano-1,2,3,4,4a,5,8,8a-octahydronaphthalene was obtained. 3,450 ppm of the compound represented by the formula [I] and 1,050 ppm of the compound represented by the formula [II] were contained in this fraction.

### 〈Example 3〉

A mixture of 600 g of the 5-ethyl-2-norbornene recovered from the reaction mixture in Example 2, 82 g of dicyclopentadiene and 88 g of methylcyclohexane, was prepared and continuous reaction was done for 5 hours in the like manner as in Example 2. 300 g of 5-ethyl-2-norbornene was recovered and 53 g of a fraction mainly containing 2-ethyl-1,4,5,8-dimethano-1,2,3,4,4a,5,8,8a-octahydronaphthalene was obtained by fractional distillation. 3,460 ppm of the compound represented by the formula [I] and 1,070 ppm of the compound represented by the formula [II] were contained in this fraction.

### 〈Comparative Example 2〉

Using a tubular reactor of 5 mm in inner diameter, a portion of 127 cm having inner volume of 25 ml was immersed into an oil bath, which was regulated to 250°C. 600 g of the 5-ethyl-2-norbornene and 1000 g of dicyclopentadiene were mixed together and the mixture was fed into the tubular reactor using a pump at space velocity of 4 h⁻¹. At a point before the heating zone, 1-butene was continuously fed, in which the molar ratio of 1-butene to dicyclopentadiene was adjusted to 1:1. The pressure in the reaction system was maintained to 5 MPa using a pressure-regulating valve. The reaction was continued for 1 hour to obtain a reaction mixture and it was subjected to fractional distillation at 0.1 kPa. Thus, 35 g of the intended fraction mainly containing 2-ethyl-1,4,5,8-dimethano-1,2,3,4,4a,5,8,8a-octahydronaphthalene was obtained. In this fraction, 5,050 ppm of the compound represented by the formula [I] and 3,150 ppm of the compound represented by the formula [II] were contained.

### 〈Comparative Example 3〉

Into 1 lit. autoclave were introduced 400 g of 5-ethyl-2-norbornene, 68 g of dicyclopentadiene and 29 g of 1-butene. The mixture was allowed to react at 300°C for 6.5 hours. The reaction mixture was subjected to fractional distillation at 1 kPa. Thus, 35 g of the intended fraction mainly containing 2-ethyl-1,4,5,8-dimethano-1,2,3,4,4a,5,8,8a-octahydronaph-thalene was obtained. In this fraction, 17,050 ppm of the compound represented by the formula [I] and 7,850 ppm of the compound represented by the formula [II] were contained.

### Industrial Applicability

The total content of cyclopentadiene turners as represented by the formulae [I] and [II] in the composition of 2-ethyl-1,4,5,8-dimethano-1,2,3,4,4a,-5,8,8a-octahydronaphthalene represented by the formula [III] according to the present invention, is limited within a specific range. Therefore, when the composition is used as a raw material for polymerization, the gelation can be avoided substantially.

## Claims

1. A 2-ethyl-1,4,5,8-dimethano-1,2,3,4,4a,5,8,8a-octahydronaphthalene composition for polymerization as represented by the following formula [III], wherein the total content of cyclopentadiene trimers as resented by the following formulae [I] and [II] is in the range of 100 to 5,000 ppm.

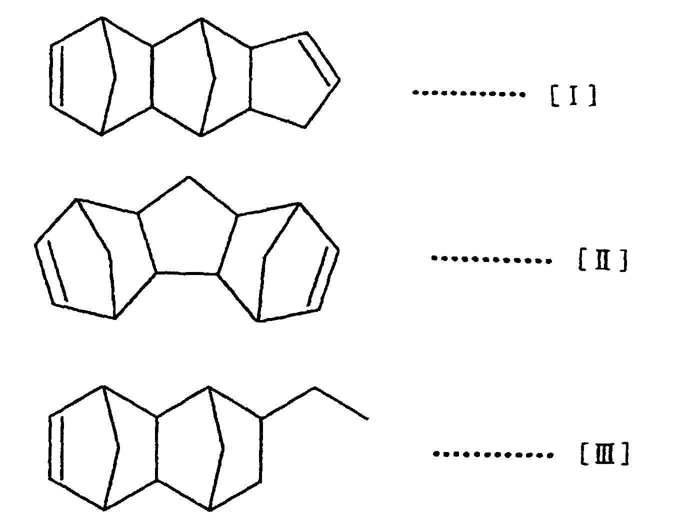

2. A composition of 2-ethyl-1,4,5,8-dimethano-1,2,3,4,4a,5,8,8a-octahydronaphthalene for polymerization represented by said formula [III], in which the content of cyclopentadiene trimer as represented by said formula [II] is in the range of 100 to 3,000.

3. A process for producing a composition of 2-ethyl-1,4,5,8-dimethano-1,2,3,4,4a,5,8,8a-octahydronaphthalene for polymerization represented by the following formula [III], in which composition the total content of cyclopentadiene trimers represented by the following formulae [I] and [II] is in the range of 100 to 5,000 ppm, said process comprising the step of reacting cyclopentadiene and/or clicyclopentadiene and 5-ethyl-2-norbornene as represented by the following formula [IV] at temperature of 100 to 350°C, pressure of 0.5 to 20 MPa and residence time of 0.1 to 360 min., in which the quantity of the 5-ethyl-2-norbornene is 1 to 20 times by mole as much as the sum of the moles of dicyclopentadiene plus one half of the moles of cyclopentadiene.

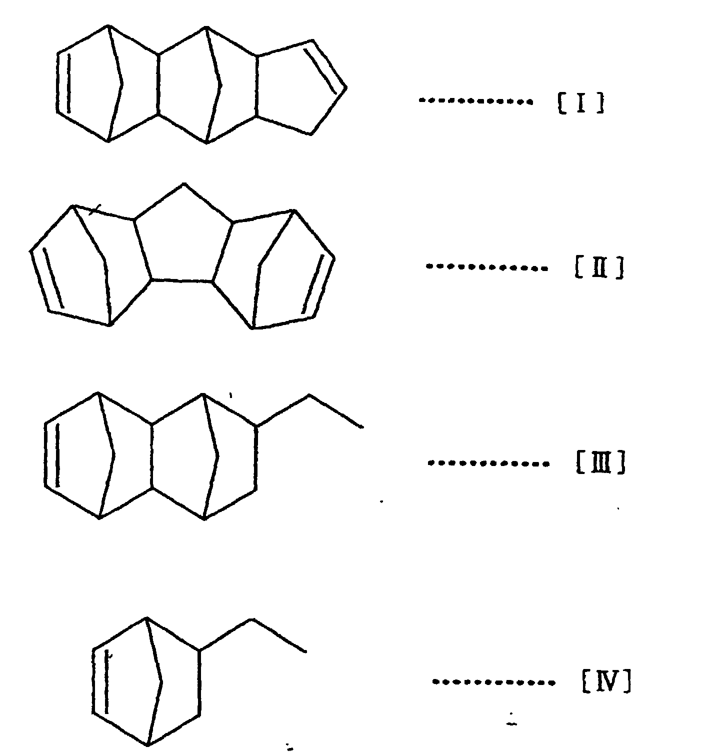

4. A process for producing a composition of 2-ethyl-1,4,5,8-dimethano-1,2,3,4,4a,5,8,8a-octahydronaphthalene for polymerization represented by said formula [III], in which composition the total content of cyclopentadiene trimers represented by said formulae [I] and [II] is in the range of 100 to 5,000 ppm, and said process comprising the step of reacting cyclopentadiene and/or dicyclopentadiene, 5-ethyl-2-norbornene as represented by said formula [IV] and 1-butene at temperature of 100 to 350°C, pressure of 0.5 to 20 MPa and residential time of 0.1 to 360 min., in which the quantity of said 1-butene is 0.1 to 10 times by mole and the quantity of said 5-ethyl-2-norbornene is 1 to 20 times by mole as much as the sum of the moles of dicyclopentadiene plus one half of the moles of cyclopentadiene.

5. A process for producing a composition of 2-ethyl-1,4,5,8-dimethano-1,2,3,4,4a,5,8,8a-octahydronaphthalene for polymerization represented by said formula [III], in which composition the total content of cyclopentadiene trimers represented by said formulae [I] and [II] is in the range of 100 to 5,000 ppm, and said process comprising the steps of: reacting cyclopentadiene and/or dicyclopentadiene, 5-ethyl-2-norbornene as represented by said formula [IV] and 1-butene at temperature of 100 to 350°C, pressure of 0.5 to 20 MPa and residential time of 0.1 to 360 min. and reusing said 5-ethyl-2-norbornene by recycling it, in which the quantity of said 1-butenen is 0.1 to 10 times by mole and the quantity of said 5-ethyl-2-norbornene is 1 to 20 times by mole as much as the sum of the moles of dicyclopentadiene plus one half of the moles of cyclopentadiene.

6. A process as claimed in any one of Claims 3 to 5, in which the content of cyclopentadiene trimer represented by said formula [II] is in the range of 100 to 3,000 ppm.
